# EUROPÄISCHE PATENTANMELDUNG

(11) **EP 3 984 522 A1**
(43) Veröffentlichungstag der Anmeldung: **20.04.2022**
(21) Anmeldenummer: 20202591.2
(22) Anmeldetag: 19.10.2020
(51) Int. Cl.: A61K 9/00, A24F 40/10, A61K 9/08, A61K 31/352, A61K 47/44, A61M 15/06

(54) **ZUSAMMENSETZUNG, VORRICHTUNG UND VERWENDUNG**

(71) Anmelder: Khaki, Shima, 40629 Düsseldorf (DE)
(72) Erfinder: Khaki, Shima, 40629 Düsseldorf (DE)
(74) Vertreter: Brinkmann & Partner

(57) **Zusammenfassung**

Die Erfindung betrifft eine Zusammensetzung zur Aufnahme durch den menschlichen Körper, bestehend aus einer cannabidiolhaltigen Komponente, einer ersten Ölkomponente und einer zweiten Ölkomponente, wobei die cannabidiolhaltige Komponente wenigstens 60 Gew.-% Cannabidiol aufweist und wobei die erste Ölkomponente aus wenigstens einem pflanzlichen Öl oder wenigstens einer daraus gewinnbaren Fraktion gebildet ist und wobei die zweite Ölkomponente aus wenigstens einem ätherischen Öl gebildet ist.

Ferner betrifft die Erfindung eine Vorrichtung zur Verdampfung von cannabidiolhaltigen Zusammensetzungen, vorzugsweise einer Zusammensetzung gemäß einem der Ansprüche 1 bis 12, mit einem Energiespeicher und/oder einem elektrischen Anschluss an eine externe Stromquelle, einem Vorratsbehälter zur Aufnahme der Zusammensetzung, einer Verdampfungseinrichtung zur Verdampfung der Zusammensetzung und einer Applikationseinheit zur Abgabe der verdampften Zusammensetzung an einen Benutzer, wobei zwischen Verdampfungseinrichtung und Applikationseinheit ein Strömungsweg für die verdampfte Zusammensetzung gebildet ist, die Vorrichtung aufweisend einen Sensor, wobei der Sensor im Strömungsweg angeordnet und dazu ausgebildet ist, wenigstens einen auf die verdampfte Zusammensetzung bezogenen physikalischen und/oder chemischen Parameter zu sensieren.

## Beschreibung

Die Erfindung betrifft eine Zusammensetzung zur Aufnahme durch den menschlichen Körper, aufweisend Cannabidiol. Ferner betrifft die Erfindung eine Vorrichtung zur Verdampfung von cannabidiolhaltigen Zusammensetzungen und eine Verwendung einer cannabidiolhaltigen Zusammensetzung.

Cannabidiol (CBD) ist aus dem Stand der Technik an sich bekannt. Es gehört zu der Gruppe der Cannabinoide. Im Unterschied zum ebenfalls zu den Cannabinoiden gehörenden Tetrahydrocannabinol (THC) entfaltet es jedoch im menschlichen Körper keine psychoaktive, bewusstseinsverändernde Wirkung.

Neueste Erkenntnisse zeigen, dass CBD bei benutzerseitiger Anwendung, insbesondere metabolischer Aufnahme, eine Vielzahl gesundheitlich positiver Wirkungen aufweist. Hierzu zählen insbesondere entkrampfende, entzündungshemmende, angstlösende und gegen Übelkeit gerichtete Wirkungen. Ferner deutet vieles darauf hin, dass CBD darüber hinaus antipsychotisch wirksam ist.

CBD wird bevorzugt aus den Blütenköpfen der weiblichen Hanfpflanze extrahiert. Während aus technischer Sicht CBD dem Grunde nach aus allen Hanfsorten gewonnen werden kann, kommen aufgrund gesetzlicher Vorschriften insbesondere speziell gezüchtete Hanfsorten mit geringem THC Anteil zum Einsatz.

CBD findet bereits als Arzneimittel, Nahrungsergänzungsmittel zur oralen Einnahme und zu kosmetischen Zwecken in Form von Ölen Verwendung.

Es hat sich jedoch gezeigt, dass CBD durch die bisherigen Darreichungsformen entweder nur unzureichend metabolisiert wird oder chemisch und pharmazeutisch aufwändig herzustellende Trägersubstanzen notwendig sind, um die Bioverfügbarkeit zu erhöhen.

Insbesondere im Bereich des THC bzw. Marihuana Konsums ist darüber hinaus die Aufnahme mittels Rauchen bekannt. Insbesondere THC wird gegenüber alternativen Darreichungsformen durch die inhalative Aufnahme vergleichsweise schnell verstoffwechselt.

Der Erfindung liegt damit die **Aufgabe** zugrunde, eine Zusammensetzung anzugeben, bei der die Bioverfügbarkeit von Cannabidiol bei dessen Aufnahme, insbesondere bei der inhalativen Aufnahme, verbessert ist.

Zur **Lösung** der Aufgabe wird mit der Erfindung eine Zusammensetzung zur Aufnahme durch den menschlichen Körper vorgeschlagen, bestehend aus einer cannabidiolhaltigen Komponente, einer ersten Ölkomponente und einer zweiten Ölkomponente, wobei die cannabidiolhaltige Komponente wenigstens 60 Gew.-% Cannabidiol aufweist und wobei die erste Ölkomponente aus wenigstens einem pflanzlichen Öl oder wenigstens einer daraus gewinnbaren Fraktion gebildet ist und wobei die zweite Ölkomponente aus wenigstens einem ätherischen Öl gebildet ist.

Es hat sich gezeigt, dass die cannabidiolhaltige Komponente, die erste Ölkomponente und die zweite Ölkomponente im Rahmen der erfindungsgemäßen qualitativen und quantitativen Merkmale in synergetischer Weise zusammenwirken. Hierdurch wird eine Verbesserung der Bioverfügbarkeit des Cannabidiols im menschlichen Körper erreicht. Insbesondere die Kombination aus einem pflanzlichen Öl und einem ätherischen Öl wirkt offenbar in dieser Hinsicht unterstützend. Dieser Vorteil scheint dabei nicht auf eine bestimmte Darreichungsform begrenzt zu sein, sondern zeigt sich sowohl bei oraler, inhalativer und topischer Applikation. Mit Bezug auf die inhalative Applikation hat sich darüber hinaus herausgestellt, dass die Aufnahme des Cannabidiols durch einen weiteren synergetischen Effekt unterstützt wird. Die erfindungsgemäße Zusammensetzung führt bei ihrer Verdampfung zu einem besonders homogenen und schwebefähigen Inhalat, wodurch die Resorption über die Mund-, Nasen- und Rachenschleimhäute sowie der Lungenbläschen verbessert wird. Dementsprechend gehen die mit der Zusammensetzung erreichbaren synergetischen Vorteile über die bekannten Einzelwirkungen der jeweiligen Komponenten hinaus und waren nicht erwartbar.

Die Komponenten der Zusammensetzung sind vorzugsweise so gewählt, dass die Zusammensetzung flüssig oder zumindest fließfähig ist. Hierdurch wird die direkte Resorption im Körper bzw. die Verdampfungseigenschaften bei einer Verdampfung in einer entsprechenden, insbesondere erfindungsgemäßen, Vorrichtung verbessert.

Gemäß einem bevorzugten Merkmal der Erfindung ist der Anteil der cannabidiolhaltigen Komponente in der Zusammensetzung an den gewünschten Einsatzbereich und den gewünschten Effekt anpassbar. Prinzipiell bevorzugt ist daher ein Anteil der cannabidiolhaltigen Komponente in der Zusammensetzung von 1 Gew.-% bis 70 Gew.-%. Im Bereich der Zusammensetzungen zur inhalativen Anwendung ist ein Anteil von 1 Gew.-% bis 25 Gew.-%, insbesondere 1 Gew.-% bis 15 Gew.-%, besonders bevorzugt. Es hat sich gezeigt, dass hierdurch die Bildung eines homogenen und stabilen Inhalats weiter verbessert wird. Im Bereich der Zusammensetzungen zur oralen Anwendung, insbesondere in Tropfenform, ist ein Anteil von 10 Gew.-% bis 40 Gew.-%, insbesondere 15 Gew.-% bis 35 Gew.-%, besonders bevorzugt. Es hat sich gezeigt, dass eine solche Zusammensetzung für eine wirksame Aufnahme über die Mundschleimhaut optimiert ist. Im Bereich der Zusammensetzungen zur topischen Anwendung ist ein Anteil von 25 Gew.-% bis 70 Gew.-%, insbesondere 35 Gew.-% bis 70 Gew.-%, besonders bevorzugt. Es hat sich gezeigt, dass ein vergleichsweise hoher Anteil der cannabidiolhaltigen Komponente bei topischen Anwendungen verbesserte Wirkung zeigt.

Erfindungsgemäß weist die Zusammensetzung eine cannabidiolhaltige Komponente auf. Vorzugsweise kann die cannabidiolhaltige Komponente neben Cannabidiol weitere Stoffe aufweisen, welche als Nebenprodukte bei der Cannabidiol-Gewinnung anfallen. Dies können insbesondere Cannabinoide, wie etwa Cannabidiolsäure (CBDa), Cannabinol (CBN), Tetrahydrocannabinol (THC), Cannabichromen (CBC) und/oder Cannabigerol (CBG) und/oder deren Derivate sein. Ferner können weitere Bestandteile der Hanfblüte, insbesondere der weiblichen Hanfblüte in der cannabidiolhaltigen Komponente enthalten sein. Diese können insbesondere durch Terpene und Flavonoide gebildet sein. Diese zusätzlichen Bestandteile können bereits in Spuren positive gesundheitliche Wirkung zeigen.

Es ist in dieser Hinsicht bevorzugt, dass die cannabidiolhaltigen Komponente bis zu 25 Gew.-% CBDa aufweist. Weiter bevorzugt sind neben CBD zwischen 1 Gew.-% und 20 Gew.-%, besonders bevorzugt zwischen 5 Gew.-% und 15 Gew.-%, CBDa in der cannabidiolhaltigen Komponente enthalten. Es hat sich herausgestellt, dass CBDa die Wirkung von CBD in einem gewissen Anteilsbereich positiv verstärkt und andererseits - ebenso wie CBD selbst - die psychoaktive Wirkung von THC abschwächt.

Prinzipiell ist es möglich, dass die erfindungsgemäße cannabidiolhaltige Komponente Tetrahydrocannabinol aufweisen kann, um gesundheitlich positive Effekte des THC in die Zusammensetzung einzubeziehen und gegebenenfalls eine gewisse psychoaktive Wirkung zu erreichen. Vorzugsweise sind jedoch nicht mehr als bis zu 30 Gew.-% Tetrahydrocannabinol insgesamt in der Zusammensetzung und nicht mehr als 50 Gew.-% in der cannabidiolhaltigen Komponente enthalten. Besonders bevorzugt ist ein THC Anteil in der Zusammensetzung von weniger als 10 Gew.-%, besonders bevorzugt von weniger als 5 Gew.-%. Es hat sich gezeigt, dass ein Anteil von unter 10 Gew.-% und insbesondere unter 5 Gew.-% THC in der Zusammensetzung nahezu keine bzw. keine psychoaktive Wirkung mehr aufweist und stattdessen die gesundheitlich positiven Wirkungen des THC in den Vordergrund treten. Anmelderseitig wird davon ausgegangen, dass dies auf die synergetische Wirkung zwischen THC, CBD und vorzugsweise CBDa zurückzuführen ist. Aufgrund unterschiedlicher nationaler gesetzlicher Bestimmungen ist es jedoch gegebenenfalls erforderlich, dass der Anteil an THC in der Zusammensetzung an rechtliche Grenzwerte angepasst ist. So macht es beispielsweise die derzeitige Rechtslage der Bundesrepublik Deutschland erforderlich, dass der Anteil an THC in der Zusammensetzung unter 0,2 Gew.-% betragen muss, um ein normal verkehrsfähiges Produkt zu erhalten. Es ist daher besonders bevorzugt, dass der Anteil an THC in der Zusammensetzung weniger als 0,2 Gew.-%, insbesondere weniger als 0,05 Gew.-%, Tetrahydrocannabinol beträgt. Gemäß einem weiteren bevorzugten Merkmal der Erfindung sind die cannabidiolhaltige Komponente und damit auch die Zusammensetzung THC frei ausgebildet. Der Begriff "THC frei" bezeichnet im Sinne der Erfindung einen THC-Anteil unterhalb der technischen und mit vertretbarem wirtschaftlichem Aufwand zu erreichenden Nachweisgrenze.

Erfindungsgemäß weist die cannabidiolhaltige Komponente wenigstens 60 Gew.-% Cannabidiol auf. Besonders bevorzugt ist ein Anteil von mehr als 75 Gew.-%, insbesondere mehr als 85 Gew.-%. Dies ist insoweit von Vorteil, da CBD den hauptsächlich metabolisch wirksamen Inhaltsstoff der Komponente ausmacht. Die übrigen optionalen Bestandteile sind entweder Nebenprodukte des Herstellungsprozesses oder entfalten Ihre Wirkung in synergetischer Kombination mit CBD, wie insbesondere CBDa und/oder THC. Ein vergleichsweise hoher Anteil an CBD in der cannabidiolhaltigen Komponente ist daher vorteilhaft. Es kann daher gemäß einem besonders bevorzugten Merkmal der Erfindung vorgesehen sein, dass die cannabidiolhaltige Komponente vollständig aus CBD gebildet sein. Das Wort "vollständig" meint im Sinne der Erfindung ein mit vertretbarem technischem und wirtschaftlichem Aufwand herstellbares Produkt. Ein solches Produkt kann noch Reste bzw. Spuren anderer Stoffe enthalten, welche sich mit technisch üblichen physikalischen sowie chemischen Trennverfahren und/oder Aufbereitungsmethoden nicht entfernen lassen. Eine vollständig aus CBD gebildete cannabidiolhaltige Komponente ist vorzugsweise ein CBD-Isolat. Ein solches CBD-Isolat weist vorzugsweise einen CBD-Gehalt von 99,0 Gew.-% bis 99,9 Gew.-%, insbesondere 99,5 Gew.-% auf. In diesem Fall entspricht der Anteil der cannabidiolhaltigen Komponente in der Zusammensetzung im Wesentlichen dem Anteil des Cannabidiols in der Zusammensetzung. "Im Wesentlichen" meint im Sinne der Erfindung damit unter Vernachlässigung der im Isolat enthaltenen Reste und/oder Spuren anderer Stoffe, welche sich mit technisch üblichen physikalischen sowie chemischen Trennverfahren und/oder Aufbereitungsmethoden nicht entfernen lassen.

Erfindungsgemäß weist die Zusammensetzung eine erste Ölkomponente und eine zweite Ölkomponente auf. Die Zusammensetzung ist daher vorzugsweise ein cannabidiolhaltiges Ölgemisch. Dabei ist erste Ölkomponente aus wenigstens einem pflanzlichen Öl oder wenigstens einer daraus gewinnbaren Fraktion gebildet, während die zweite Ölkomponente aus wenigstens einem ätherischen Öl gebildet ist. Dabei kann die erste Ölkomponente aus einem einzigen pflanzlichen Öl, aus mehreren pflanzlichen Ölen, aus einer aus dem jeweiligen pflanzlichen Öl gewinnbaren Fraktion oder mehreren Fraktionen gebildet sein. Auch die zweite Ölkomponente kann aus einem einzigen ätherischen Öl oder auch mehreren ätherischen Ölen gebildet sein.

Ein "pflanzliches Öl" bezeichnet im Sinne der Erfindung ein aus Ölpflanzen gewonnenes fettes Öl (Lipid). Im Gegensatz zu den ätherischen Ölen, die kein Fett enthalten, verdampfen pflanzliche Öle rückstandsfrei. "Ätherische Öle" bezeichnet im Sinne der Erfindung leicht flüchtige und häufig leicht entzündbare Stoffgemische, die aus verschiedenen ineinander löslichen, organischen Stoffen wie Alkoholen, Estern, Ketonen und/oder Terpenen bestehen. Sie werden synthetisch oder aus natürlichen Quellen durch Wasserdampfdestillation, Extraktion oder Auspressen der Pflanzen oder der Pflanzenteile gewonnen. Ätherische Öle werden häufig in den Blättern von Pflanzen produziert und im Pflanzen-Gewebe gespeichert. Im Gegensatz zu pflanzlichen Ölen verdunsten ätherische Öle rückstandslos.

Eine "aus einem pflanzlichen Öl gewinnbare Fraktion" bezeichnet im Sinne der Erfindung eine Teilmenge einer in einem Öl insgesamt vorliegenden Gesamtmenge an Triglyceriden bestimmter Kettenlänge. Vorzugsweise sind dies SCT-Öle, MCT-Öle und/oder LCT-Öle. "SCT" steht dabei für "Short Chain Triglycerides", während "MCT" für "Medium Chain Triglycerides" und "LCT" für "Long Chain Triglycerides" steht. Vorzugsweise werden dabei MCT-Öle und/oder LCT-Öle verwendet. Denkbar ist jedoch auch die Verwendung reiner SCT-Öle, welche insbesondere im Bereich der oralen Anwendung der Zusammensetzung aufgrund der geringen Viskosität von Vorteil ist. MCT-Öle finde besonders bevorzugt Verwendung im Bereich der Inhalate, da diese über dahingehend vorteilhafte Rauchpunkte verfügen. "Inhalat" im Sinne der Erfindung bezeichnet grundsätzlich Gase und/oder Aerosole, die über Mund und/oder Nase inhalierbar sind. Insbesondere ist damit jedoch die erfindungsgemäße Zusammensetzung in ihrer mittels eines Vaporizers verdampften Form gemeint.

Ferner kommen Mischungen aus SCT-Ölen mit MCT- oder LCT-Ölen in Betracht. Sofern die Zusammensetzung zur Herstellung eines Inhalats verwendet werden soll, lassen sich hierdurch bevorzugte Rauchpunkte einstellen. LCT-Öle kommen dabei aufgrund ihrer vergleichsweise hohen Viskosität insbesondere in den Fällen zum Einsatz, in denen die Zusammensetzung als Massageöl bei topischer Applikation Verwendung finden soll.

SCT-Öle bestehen aus einer Gruppe von Triglyceriden, die kurzkettige gesättigte Fettsäuren mit Kettenlängen von C1 bis C5 enthalten. Zu den kurzkettigen Tryglyceriden zählen insbesondere Triacetin und Tributyrin.

MCT-Öle bestehen aus einer Gruppe von Triglyceriden, die mittelkettige gesättigte Fettsäuren mit Kettenlängen von C6 bis C12 enthalten. Zu den mittelkettigen Fettsäuren zählen die Capron- (C6), Capryl- (C8), Caprin- (C10) und die Laurinsäure (C12). Es handelt sich dabei um gesättigte Fettsäuren, welche vor allem in tropischen Pflanzenfetten wie Kokosfett (ca. 60 %) und Palmkernöl (ca. 55 %) vorkommen. Eine mögliche Zusammensetzung eines MCT-Öls ist insbesondere ist 50-80 Gew.-% Caprylsäure, 25-45 Gew.-% Caprinsäure, Laurinsäure bis zu 3 Gew.-% und bis zu 2 Gew.-% Capronsäure.

LCT-Öle bestehen demgegenüber aus einer Gruppe von Triglyceriden, die langkettige gesättigte Fettsäuren mit Kettenlängen von C13 bis C21 enthalten. Zu den langkettigen Fettsäuren gehören insbesondere die Myristin- (C14), Palmitin- (C16) und die Stearinsäure (C18).

Prinzipiell ist ein Anteil der ersten Ölkomponente in der Zusammensetzung von 1 Gew.-% bis 98 Gew.-% möglich. Es hat sich gezeigt, dass vorzugsweise wenigstens 15 Gew.-% der ersten Ölkomponente in der Zusammensetzung enthalten sein sollten, um eine für alle Anwendungen ausreichende Einstellung der Viskosität und der Flüchtigkeit der Zusammensetzung vornehmen zu können. Ferner ist prinzipiell ein Anteil der zweiten Ölkomponente in der Zusammensetzung von 1 Gew.-% bis 70 Gew.-% möglich. Unterhalb des unteren Grenzwertes sind die Wirkungen der zweiten Ölkomponente zu gering, um den erfindungsgemäßen Effekt zu erreichen. Oberhalb des oberen Grenzwertes neigen die Zusammensetzungen dazu zu flüchtig zu sein. Je nach Anwendungsbereich kann es dabei vorteilhaft sein, dass der Anteil der ersten Ölkomponente in der Zusammensetzung höher oder niedriger ist als der Anteil der zweiten Ölkomponente in der Zusammensetzung. Auch Ausgestaltungen, in denen die erste und die zweite Ölkomponente mit gleichem Anteil in Zusammensetzung vorliegen sind in bestimmten Fällen bevorzugt. Je nach Anwendungsbereich haben sich bestimmte Anteile der ersten und zweiten Ölkomponente als besonders vorteilhaft erwiesen.

So ist es bei Zusammensetzungen für die inhalativen Anwendung bevorzugt, dass die erste Ölkomponente in größerem Anteil in der Zusammensetzung vorliegt, als die zweite Ölkomponente. Insbesondere bevorzugt ist damit ein Anteil der ersten Ölkomponente in der Zusammensetzung von 50 Gew.-% bis 98 Gew.-%, insbesondere 75 Gew.-% bis 98 Gew.-%. Demgegenüber ist ein Anteil der zweiten Ölkomponente in der Zusammensetzung von 1 Gew.-% bis 49 Gew.-%, insbesondere 1 Gew.-% bis 24 Gew.-%, bevorzugt. Es hat sich gezeigt, dass hierdurch die Bildung eines homogenen und stabilen Inhalats weiter verbessert wird.

Bei Zusammensetzungen für die orale Anwendung ist es bevorzugt, dass die erste Ölkomponente in geringerem Anteil in der Zusammensetzung vorliegt, als die zweite Ölkomponente. Insbesondere bevorzugt ist damit ein Anteil der ersten Ölkomponente in der Zusammensetzung von 15 Gew.-% bis 40 Gew.-%, insbesondere 15 Gew.-% bis 35 Gew.-%. Demgegenüber ist ein Anteil der zweiten Ölkomponente in der Zusammensetzung von 45 Gew.-% bis 70 Gew.-%, insbesondere 50 Gew.-% bis 70 Gew.-%, bevorzugt. Es hat sich gezeigt, dass eine solche Zusammensetzung für eine wirksame Aufnahme über die Mundschleimhaut optimiert ist.

Bei Zusammensetzungen für die topische Anwendung können die erste und die zweite Ölkomponente in verschiedenem oder gleichem Verhältnis in der Zusammensetzung enthalten sein. Es ist bevorzugt, dass die erste Ölkomponente in höherem oder zumindest gleichem Anteil in der Zusammensetzung vorliegt als die zweite Ölkomponente. Insbesondere bevorzugt ist damit ein Anteil der ersten Ölkomponente in der Zusammensetzung von 15 Gew.-% bis 65 Gew.-%, insbesondere 20 Gew.-% bis 55 Gew.-%. Demgegenüber ist ein Anteil der zweiten Ölkomponente in der Zusammensetzung von 10 Gew.-% bis 25 Gew.-%, insbesondere 10 Gew.-% bis 20 Gew.-%, bevorzugt. Es hat sich gezeigt, dass eine solche Zusammensetzung bei topischen Anwendungen verbesserte Wirkung zeigt.

Gemäß einem bevorzugten Merkmal der Erfindung ist vorgesehen, dass das pflanzliche Öl ausgewählt ist aus der Gruppe Palmöl, Sojaöl, Rapsöl, Sonnenblumenöl, Hanföl, Palmkernöl, Baumwollsamenöl, Erdnussöl, Maiskeimöl, Kokosnussöl, Olivenöl, Sesamöl, Leinöl oder Diestelöl, Pinienkernöl, Haselnussöl, Macadamiaöl, Pekanussöl, Walnussöl, Kürbiskernöl, Mandelkernöl, Traubenkernöl oder einer Mischung daraus. Diese pflanzlichen Öle haben sich unabhängig von der Verwendung als vorteilhaft erwiesen. Besonders bevorzugt sind Olivenöl, Hanföl, Sojaöl und Erdnussöl.

Gemäß einem besonders bevorzugten Merkmal der Erfindung sind mit Blick auf die Bildung eines Inhalats, Mischungen aus MCT-Öl, insbesondere eines aus Kokosnussöl gewonnenen MCT-Öls, und einem oder zwei pflanzlichen Öl, insbesondere der obigen pflanzlichen Öle, von Vorteil. Bevorzugt ist eine Mischung aus MCT-Öl und einem oder zwei Ölen der Gruppe Olivenöl, Erdnussöl, Hanföl und Sojaöl. Vorzugsweise betragen die Mischungsverhältnisse in einer zweiteiligen Mischung aus MCT/Öl von 3:1 bis 1:3. Vorzugsweise betragen die Mischungsverhältnisse in einer dreiteiligen Mischung aus MCT/1.Öl/2.Öl von 6:1:1 bis 1:1:6 und bis 1:6:1. Besonders bevorzugt ist eine Mischung aus MCT/Olivenöl im Verhältnis 3:1 oder MCT/Hanföl im Verhältnis 3:1 oder MCT/Erdnussöl im Verhältnis 2:1 oder MCT/Sojaöl im Verhältnis 1:1. Besonders bevorzugt ist ferner eine Mischung aus MCT/Olivenöl/Sojaöl im Verhältnis 6:1:1 oder MCT/Olivenöl/Hanföl im Verhältnis 5:2:1 oder MCT/Olivenöl/Erdnussöl im Verhältnis 5:2:1 oder MCT/Sojaöl/Hanföl im Verhältnis 4:2:2. Vorgenannte Mischungen haben sich insbesondere mit Bezug auf die inhalative und orale Anwendung als besonders bevorzugt erwiesen. Insbesondere der Rauchpunkt dieser Mischungen sind dahingehend optimiert. Selbstverständlich sind jedoch auch Mischungen aus pflanzlichen Ölen ohne MCT-Öl möglich und bevorzugt. Insbesondere Mischungen Olivenöl und Sojaöl, Olivenöl und Hanföl, Sojaöl und Erdnussöl, Hanföl und Erdnussöl, Olivenöl, Sojaöl und Erdnussöl oder Sojaöl, Hanföl und Erdnussöl haben sich als vorteilhaft erwiesen.

Erfindungsgemäß ist die zweite Ölkomponente aus wenigstens einem ätherischen Öl gebildet. Gemäß einem bevorzugten Merkmal der Erfindung weist das ätherische Öl Terpene, insbesondere Monoterpene und/oder Sesquiterpene, und/oder aromatische Verbindungen auf. Diese Verbindungen haben sich insbesondere hinsichtlich der Bildung eines stabilen und homogen verteilten Inhalats, als auch bei der Resorption während der oralen Aufnahme als positiv erwiesen.

Gemäß einem bevorzugten Merkmal der Erfindung ist vorgesehen, dass das ätherische Öl ausgewählt ist aus der Gruppe Eukalyptusöl, Teebaumöl, Rosmarinöl, Thymianöl, Pfefferminzöl, Lavendelöl, Kampferöl, Kamillenöl, vorzugsweise auf Basis Römischer Kamille oder Echter Kamille, Grapefruitöl, Bergamotteöl, Weihrauchöl, Majoranöl, Orangenöl, Mandarinenöl, Grün-Minzöl, Ingweröl, Pfefferöl, Zitronenöl, Limettenöl, Basilikumöl, Pinienöl, Bittermandelöl oder ätherisches Hanföl. Neben den an sich bekannten physiologischen Wirkungen der einzelnen Öle, verbessern diese Öle darüber hinaus in synergetischer Wirkung mit der ersten Ölkomponente und der cannabidiolhaltigen Komponente wie vorstehend beschrieben die Wirkweise der Zusammensetzung an sich. Besonders bevorzugt sind ätherische Öle der Gruppe Eukalyptusöl, Teebaumöl, Rosmarinöl, Thymianöl, Pfefferminzöl, Kamillenöl, Ingweröl, Orangenöl, Bittermandelöl, ätherisches Hanföl, Kümmelöl, Kurkumaöl, Rosenöl, Jasminöl, Fenchelöl, Anisöl, Kakaobohnenöl, Myrrheöl.

Nachfolgend werden Beispiele für bevorzugte Zusammensetzungen gegeben
Zusammensetzung 1

| | |
|---|---|
| CBD | 20 Gew.-% |
| MCT-Öl | 25 Gew.-% |
| Olivenöl | 25 Gew.-% |
| Bittermandelöl | 15 Gew.-% |
| Orangenöl | 10 Gew.-% |
| Kamillenöl | 5 Gew.-% |

Zusammensetzung 2

| | |
|---|---|
| CBD | 70 Gew.-% |
| Sojaöl | 15 Gew.-% |
| Traubenkernöl | 5 Gew.-% |
| Kurkumaöl | 5 Gew.-% |

Zusammensetzung 3

| | |
|---|---|
| CBD | 30 Gew.-% |
| CBDa | 10 Gew.-% |
| THC | 10 Gew.-% |
| Erdnussöl | 5 Gew.-% |
| Hanföl | 25 Gew.-% |
| Myrrheöl | 5 Gew.-% |
| Thymianöl | 10 Gew.-% |

Zusammensetzung 4

| | |
|---|---|
| CBD | 35 Gew.-% |
| CBDa | 15 Gew.-% |
| Erdnussöl | 5 Gew.-% |
| Hanföl | 25 Gew.-% |
| Myrrheöl | 5 Gew.-% |
| Thymianöl | 10 Gew.-% |

Zusammensetzung 5

| | |
|---|---|
| CBD | 5 Gew.-% |
| Kürbiskernöl | 5 Gew.-% |
| Hanföl | 25 Gew.-% |
| Sojaöl | 25 Gew.-% |
| Zitronenöl | 10 Gew.-% |
| Bergamotteöl | 10 Gew.-% |
| Ätherisches Hanföl | 10 Gew.-% |
| Ingweröl | 10 Gew.-% |

Obgleich die Anteile der cannabidiolhaltigen Komponente, sowie der beiden Ölkomponenten hinsichtlich der verschiedenen Anwendungsbereiche auf einander abgestimmt sind, sind die erfindungsgemäßen Zusammensetzungen für alle Anwendungsbereiche dem Grunde nach geeignet und bestimmt.

Die Erfindung betrifft darüber hinaus verschiedene Verwendungen für die erfindungsgemäße Zusammensetzung. Insbesondere eine Verwendung zur Herstellung eines Inhalats. Hierbei handelt es sich um eine Verwendung der Zusammensetzung als flüssiger Vaporizer-Wirkstoff. Darüber hinaus kann die erfindungsgemäße Zusammensetzung als Tinktur zur Applikation auf Haut, Nägeln und/oder Haaren, als Körperöl, insbesondere Massageöl, als Tropfen zur oralen Aufnahme, als Wirkstoff für Nahrungsergänzungsmittel- und/oder Arzneistoffkapseln zur oralen oder rektalen Applikation oder als Bestandteil von Duftträgern, insbesondere von Dufthölzern, von Duftbäumen für Fahrzeuge oder von Räucherstäbchen verwendet werden.

Die Erfindung betrifft ferner eine Vorrichtung zur Verdampfung von cannabidiolhaltigen Zusammensetzungen, vorzugsweise einer erfindungsgemäßen Zusammensetzung, mit einem Energiespeicher und/oder einem elektrischen Anschluss an eine externe Stromquelle, einem Vorratsbehälter zur Aufnahme der Zusammensetzung, einer Verdampfungseinrichtung zur Verdampfung der Zusammensetzung und einer Applikationseinheit zur Abgabe der verdampften Zusammensetzung an einen Benutzer, wobei zwischen Verdampfungseinrichtung und Applikationseinheit ein Strömungsweg für die verdampfte Zusammensetzung gebildet ist, die Vorrichtung aufweisend einen Sensor, wobei der Sensor im Strömungsweg angeordnet und dazu ausgebildet ist, wenigstens einen auf die verdampfte Zusammensetzung bezogenen physikalischen und/oder chemischen Parameter zu sensieren.

Es können mehr als ein Sensor und/oder ein Sensor mit verschiedenen Funktionen vorgesehen sein. Der Sensor ist insbesondere dazu ausgebildet, physikalische und/oder chemische Parameter zu erfassen. Diese Parameter sind insbesondere Volumenstrom der verdampften Zusammensetzung, Konzentration wenigstens eines chemischen Bestandteils der verdampften Zusammensetzung, Partialdruck wenigstens eines chemischen Bestandteils der verdampften Zusammensetzung und/oder Temperatur der verdampften Zusammensetzung.

Obgleich die Erfassung von Parametern der Zusammensetzung im verdampften Zustand für sich genommen bereits ausreichend sind, um eine vorteilhafte Steuerung des Verdampfungsprozesses vornehmen zu können, ist es darüber hinaus von Vorteil, Parameter betreffend die unverdampfte Zusammensetzung zu erfassen. Zu diesem Zweck ist es vorzugsweise vorgesehen, wenigstens einen Sensor in einem mit der Zusammensetzung befüllbaren Vorratsbehälter der Vorrichtung anzuordnen. Dieser Sensor ist vorzugsweise dazu ausgebildet, wenigstens einen auf die unverdampfte Zusammensetzung bezogenen physikalischen und/oder chemischen Parameter zu sensieren. Diese Parameter sind insbesondere Füllstand der unverdampften Zusammensetzung im Vorratsbehälter, Konzentration wenigstens eines chemischen Bestandteils der unverdampften Zusammensetzung und/oder Temperatur der verdampften Zusammensetzung. Die Erfassung dieser zusätzlichen Parameter erlaubt eine genauere Steuerung bzw. Regelung des Verdampfungsprozesses, was im Ergebnis zu einem besonders homogenen und stabilen Inhalats beiträgt.

Es hat sich gezeigt, dass erfindungsgemäße Zusammensetzungen, insbesondere in der besonderen Ausgestaltung als Vaporizer-Wirkstoff, in einer erfindungsgemäßen Vorrichtung besonders homogen verdampft werden können und damit zur Bildung eines homogenen und stabilen Inhalats beitragen. Der Verdampfungsvorgang ist damit insbesondere durch die Sensorik erfassbar und regelbar. Ferner können die Sensordaten dazu genutzt werden, bestimmte auf die verdampfte Zusammensetzung bezogene Parameter zu visualisieren. Dies kann insbesondere der Füllstand eines Behälters zur Aufnahme der Zusammensetzung, die seit der Befüllung abgegebene Menge an Inhalat, insbesondere angegeben in "Zügen" oder "Puffs", die während des aktuellen Betriebs der Vorrichtung an den Benutzer abgegebene Art oder Menge eines der Bestandteile der Zusammensetzung, wie etwa Menge an aufgenommenem CBD, CBDa und/oder THC insgesamt oder pro Abgabeeinheit. Ferner besteht natürlich darüber hinaus die Möglichkeit, Volumenstrom des entstehenden Inhalats mittels der Sensorik zu messen und die Verdampfungseinrichtung nach Vorbild einer SOLL/IST Schaltung zu regeln, um die gewünschte Abgabemenge der Zusammensetzung insgesamt oder einzelner Bestandteile der Zusammensetzung einzustellen.

Es ist daher gemäß einem bevorzugten Merkmal der Erfindung eine Rechnereinheit und eine Sendeeinrichtung zur, insbesondere kabellosen, Datenübertragung vorgesehen, wobei die Rechnereinheit mit dem Sensor und der Sendeeinrichtung datentechnisch verbunden ist und dazu ausgebildet ist, Sensordaten datentechnisch zu verarbeiten und die derart verarbeiteten Sensordaten an die Sendeeinrichtung zum Zwecke der Übertragung an eine korrespondierende Empfangseinrichtung weiterzuleiten. Hierdurch lassen sich die Sensordaten vorzugsweise an eine externe Datenverarbeitungseinrichtung, wie insbesondere ein Mobilfunkgerät, einen Laptop oder einen PC, senden. Die Sendeeinrichtung ist vorzugsweise als Funksendeeinrichtung ausgebildet. Bevorzugt dient sie der Übertragung von Daten mittels WLAN, Bluetooth und/oder NFC. Alternativ oder zusätzlich ist jedoch auch eine kabelgestützte Übertragung, insbesondere via USB, insbesondere USB-C, Verbindung möglich. Die Sendeeinrichtung stellt hierzu vorzugsweise einen entsprechenden Anschluss bereit. Dieser Anschluss kann vorzugsweise ebenfalls dazu benutzt werden, einen Energiespeicher der Vorrichtung, insbesondere einen Akkumulator aufzuladen.

Die Sensordaten können dabei entweder bereits von der internen Rechnereinheit der Vorrichtung visualisierungsbereit aufbereitet worden sein. Alternativ können die Rohdaten der Sensorik übermittelt werden und von der externen Datenverarbeitungseinrichtung entsprechend weiterverarbeitet, insbesondere ausgewertet, und über eine Ausgabeeinheit, wie etwa ein Display, der Datenverarbeitungseinrichtung ausgegeben werden. Besonders bevorzugt ist die Datenverarbeitungseinrichtung ein Smartphone. Mittels einer entsprechenden App werden die empfangenen Sensordaten ausgewertet und mittels des Displays dem Benutzer angezeigt. Hierdurch

Die Erfindung betrifft insbesondere die Verwendung einer erfindungsgemäßen Zusammensetzung zur Herstellung eines Inhalats mittels einer Vorrichtung gemäß einem der Erfindung. Bei der Vorrichtung gemäß der Erfindung handelt es sich bevorzugt um einen tragbaren Vaporizer mit aufladbarem Akkumulator als Energiespeicher.

Gemäß einer bevorzugten Ausgestaltung der Erfindung weist die Vorrichtung, insbesondere der Vaporizer ein Gehäuse zur Aufnahme des Energiespeichers und/oder des Stromanschlusses und/oder der Rechnereinheit auf.

Die Verdampfungseinrichtung dient erfindungsgemäß der Erzeugung eines Inhalats aus einer Zusammensetzung durch verdampfen. Sie weist zu diesem Zweck ein Heizelement auf. Dieses kann aus einem keramischen Material gebildet sein. Ferner ist die Verdampfungseinrichtung mit dem Vorratsbehälter zum Zwecke der Versorgung mit der Zusammensetzung strömungstechnisch verbunden. Verdampfungseinrichtung und Vorratsbehälter können separate Bauteile sein, die durch eine Leitung miteinander verbunden sind. Alternativ können Verdampfungseinrichtung und Vorratsbehälter integriert ausgebildet sein. Besonders bevorzugt Weist die Verdampfungseinrichtung den Vorratsbehälter auf, wobei das Heizelement im Vorratsbehälter angeordnet ist. Nach dieser Ausgestaltung kann auch der Sensor zur Erfassung der auf die verdampfte Zusammensetzung bezogenen Parameter im Vorratsbehälter oberhalb eines maximalen Füllpegels angeordnet sein. Alternativ ist der Sensor in einem sich an die den Vorratsbehälter oder die Verdampfungseinrichtung anschließenden separaten Strömungskanal angeordnet.

Erfindungsgemäß weist die Vorrichtung eine Applikationseinheit auf. Diese kann in Form eines Nutzabschnitts eines Mundstücks ausgebildet sein. Das Mundstück kann lösbar mit dem Gehäuse verbunden sein. Die Applikationseinheit, insbesondere der Nutzabschnitt wird bei bestimmungsgemäßem Betrieb in den Mund des Benutzers eingeführt. Im Gehäuse ist vorzugsweise eine Desinfektionseinheit angeordnet. Die Desinfektionseinheit weist vorzugsweise eine UV-Quelle zur Oberflächendesinfektion mittels UV-Strahlung auf. Das Mundstück kann bei Nichtgebrauch der Vorrichtung in eine Desinfektionsstellung überführt werden. In der Desinfektionsstellung ist der Nutzabschnitt derart im Gehäuse, insbesondere in einem Desinfektionsabschnitt der Desinfektionseinheit, angeordnet, dass es durch die Desinfektionseinheit mit UV-Strahlung bestrahlbar ist. Das Mundstück kann dadurch in vergleichsweise kurzer Zeit, beispielsweise zwischen 10 und 30 Sekunden desinfiziert werden. Die Verwendung der Vorrichtung durch mehr als eine Person ist dadurch in einfacher Weise möglich, ohne, dass das Risiko für Infektionen besteht. Insbesondere in Zeiten globaler Pandemien ist dies von großem Vorteil.

Gemäß einem bevorzugten Merkmal der Erfindung können Verdampfungseinrichtung und/oder Vorratsbehälter wenigstens teilweise im Mundstück angeordnet sein. In dieser Ausgestaltung stellt das Gehäuse vorzugsweise einen befestigungstechnischen und leitungstechnischen Anschluss zur Verbindung mit dem Mundstück bereit.

Die Desinfektionseinheit ist vorzugsweise steuerungstechnisch mit der Rechnereinheit zum Zwecke der Steuerung oder Regelung verbunden. Vorzugsweise ist ein Sensor vorgesehen, der den Desinfektionsstatus des Mundschutzes sensiert. Insbesondere ist der Sensor dafür ausgebildet, Keimkonzentrationen, insbesondere Keime in Form von Bakterien und Viren, zu messen und mit einem SOLL-Wert zu vergleichen. Sofern die Keimkonzentration nicht dem in einem Datenspeicher der Rechnereinheit hinterlegten SOLL-Wert entspricht, wird die Desinfektionsdauer und/oder die Strahlungsintensität über die Rechnereinheit erhöht. Ist der SOLL-Wert demgegenüber erreicht, wird der Desinfektionsvorgang beendet.

Vorzugsweise weist die Vorrichtung eine mit der Rechnereinheit steuerungstechnisch verbundene Signaleinrichtung auf, welche mit der Desinfektionsstatus einem Benutzer signalisiert werden kann. Hierzu weist die Signaleinheit visuelle, taktile und/oder auditive Signalelemente auf. Als visuelle Signalelemente kommen einfache Leuchtmittel und/oder ein Display in Betracht. Ein Display ist vorzugsweise in der Wand des Gehäuses angeordnet.

Grundsätzlich ist die erfindungsgemäße Vorrichtung als ortsfestes oder mobiles Verdampfungsgerät einsetzbar. Ortsfeste vergleichsweise große Vorrichtungen mit vergleichsweise großem maximalem Volumenstrom können prinzipiell zur Bedampfung von Räumen eingesetzt werden. Sie können hierzu als Stand-Alone-Unit ausgebildet sein oder in ein Lüftungssystem integriert werden.

Mobile Vorrichtungen können insbesondere in Form von tragbaren, hochmobilen Vaporizern oder in Form semi-mobiler Verdampfungsgeräte, insbesondere nach Art einer Wasserpfeife, ausgebildet sein. Semi-mobile Verdampfungsgeräte bieten einen guten Kompromiss aus maximalem Volumenstrom und Mobilität. Darüber hinaus bieten Sie die Möglichkeit, von mehreren Benutzern simultan benutzt zu werden. Es kann hierfür vorgesehen sein, dass die Vorrichtung eine Mehrzahl von Mundstücken zur Abgabe von Inhalat an jeweils einen Benutzer aufweist. Eine solche Vorrichtung weist vorzugsweise ein Vorratsbehältervolumen von 10mL bis 500mL, vorzugsweise 20mL bis 100mL, auf. Das Gewicht dieser Ausgestaltung beträgt vorzugsweise zwischen 0,5 kg und 10 kg, insbesondere zwischen 0,5 kg und 5 kg

Die hochmobile Vorrichtung, insbesondere in Form des Vaporizers weist vorzugsweise Außenmaße von 5 bis 15 cm Länge, 0,5 bis 5 cm Dicke und 1 bis 5 cm Breite auf. Der Vorratsbehälter weist vorzugsweise ein Volumen von 1mL bis 10mL, insbesondere 1mL bis 5mL, auf. Die Vorrichtung weist dabei vorzugsweise ein Gewicht von weniger als 200 g auf. Hierdurch wird sichergestellt, dass die Vorrichtung, insbesondere in der Ausgestaltung als Vaporizer, einfach manuell gehandhabt und mitgeführt werden kann.

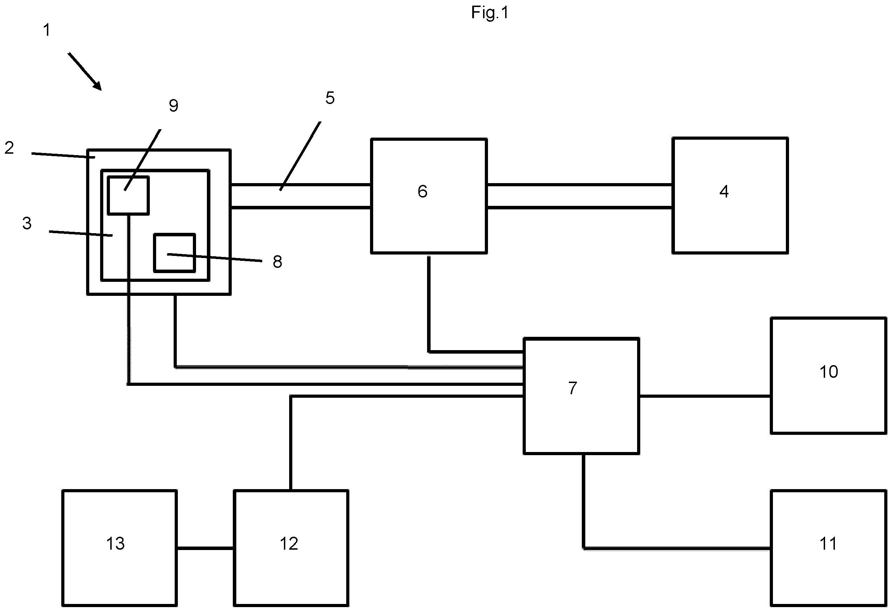

Nachfolgend wird die erfindungsgemäße Vorrichtung am Beispiel eines Vaporizers erläutert. Das Ausführungsbeispiel ist indes für den Fachmann nicht beschränkend zu verstehen. Dabei zeigt
- Fig.1: einen erfindungsgemäßen Vaporizer in schematischer Diagrammdarstellung.

Figur 1 zeigt einen erfindungsgemäßen Vaporizer 1 zur Verdampfung einer erfindungsgemäßen Zusammensetzung, insbesondere eines Vaporizer-Wirkstoffes. Der Vaporizer 1 weist eine Verdampfungseinrichtung 2 zur Verdampfung der in einem Vorratsbehälter 3 angeordneten Zusammensetzung auf. Der Vorratsbehälter 3 ist vorliegend als Teil der Verdampfungseinrichtung 2 ausgebildet. Zu erkennen ist ferner ein Heizelement 8, welches der Erhitzung der unverdampften Zusammensetzung auf eine Temperatur dient, bei der die Zusammensetzung nahezu vollständig verdampft.

Verdampfungseinrichtung 2 und Vorratsbehälter 3 sind vorliegend über einen Strömungsweg 5 mit einer Applikationseinheit 4 in Form eines Nutzabschnitts eines nicht dargestellten Mundstücks. Im Strömungsweg 5 ist ein Sensor 6 angeordnet, der dazu ausgebildet ist, wenigstens einen auf die verdampfte Zusammensetzung bezogenen physikalischen und/oder chemischen Parameter zu sensieren.

Ferner ist ein weiterer Sensor 9 im Vorratsbehälter 3 angeordnet, welcher dazu ausgebildet ist, wenigstens einen auf die unverdampfte Zusammensetzung bezogenen physikalischen und/oder chemischen Parameter zu sensieren.

Die Sensoren 6, 9 sind mit einer Rechnereinheit 7 datentechnisch verbunden. Die Rechnereinheit 7 dient unter anderem der Datenverarbeitung der empfangenen Sensordaten. Die Rechnereinheit 7 ist mit einer Sendeeinrichtung 10 zur kabellosen Datenübertragung verbunden. Die Sendeeinrichtung 10 ist als Funksendeeinrichtung ausgebildet. Vorliegend ist sie für die Übertragung von Daten mittels Bluetooth und/oder NFC an eine externe Empfangseinrichtung, insbesondere eines Smartphones, ausgerüstet.

Die Vorrichtung 1 weist ferner eine Desinfektionseinheit 11 zur UV-Desinfektion der Applikationseinheit 4 in einer Nichtgebrauchsstellung auf. Zur Detektion von Keimkonzentrationen weist die Desinfektionseinheit vorzugsweise einen weiteren, nicht dargestellten Sensor auf. Die Desinfektionseinheit 11 ist mit der Rechnereinheit 7 zu Steuerungs- und/oder Regelungszwecken datentechnisch verbunden.

Die Vorrichtung 1 weist ferner eine Signaleinrichtung 12 zur Signalisierung verschiedener Vorrichtungszustände auf. Hierzu verfügt sie über visuelle, taktile und auditive Signalmittel 13. Ein visuelles Signalmittel 13 ist vorliegend in Form eines Displays ausgebildet. Die Signaleinrichtung 12 kann insbesondere einen Ladungszustand eines nicht dargestellten Energiespeichers, insbesondere Akkumulators, vorzugsweise Lithium-Ionen-Akkumulator, einen Desinfektionsstatus der Applikationseinheit 4 und verschiedene Sensordaten der Sensoren 6, 9 visuell darstellen. Zu diesem Zweck ist die Signaleinrichtung 12 datentechnisch mit der Rechnereinheit 7 verbunden.

### Bezugszeichen

- 1: Vaporizer
- 2: Verdampfungseinrichtung
- 3: Vorratsbehälter
- 4: Applikationseinheit
- 5: Strömungsweg
- 6: Sensor
- 7: Rechnereinheit
- 8: Heizelement
- 9: Sensor
- 10: Sendeeinrichtung
- 11: Desinfektionseinheit
- 12: Signaleinrichtung
- 13: Signalmittel

## Patentansprüche

1. Zusammensetzung zur Aufnahme durch den menschlichen Körper, bestehend aus einer cannabidiolhaltigen Komponente, einer ersten Ölkomponente und einer zweiten Ölkomponente, wobei die cannabidiolhaltige Komponente wenigstens 60 Gew.-% Cannabidiol aufweist und wobei die erste Ölkomponente aus wenigstens einem pflanzlichen Öl oder wenigstens einer daraus gewinnbaren Fraktion gebildet ist und wobei die zweite Ölkomponente aus wenigstens einem ätherischen Öl gebildet ist.

2. Zusammensetzung nach Anspruch 1, **dadurch gekennzeichnet, dass** der Anteil der cannabidiolhaltigen Komponente in der Zusammensetzung 1 Gew.-% bis 70 Gew.-% beträgt.

3. Zusammensetzung nach Anspruch 2, **dadurch gekennzeichnet, dass** der Anteil an der ersten Ölkomponente in der Zusammensetzung 15 Gew.-% bis 98 Gew.-% beträgt.

4. Zusammensetzung nach Anspruch 3, **dadurch gekennzeichnet, dass** der Anteil der zweiten Ölkomponente 1 Gew.-% bis 70 Gew.-% beträgt.

5. Zusammensetzung nach Anspruch 4, **dadurch gekennzeichnet, dass** die cannabidiolhaltige Komponente bis zu 25 Gew.-% Cannabidiolsäure aufweist.

6. Zusammensetzung nach Anspruch 5, **dadurch gekennzeichnet, dass** die cannabidiolhaltige Komponente weniger als 0,2 Gew.-% Tetrahydrocannabinol aufweist

7. Zusammensetzung nach einem der Ansprüche 1 bis 6, **dadurch gekennzeichnet, dass** die erste Ölkomponente aus wenigstens einem weiteren pflanzlichen Öl oder wenigstens einer weiteren Fraktionen des weiteren pflanzlichen Öls gebildet ist und/oder dass die zweite Ölkomponente aus wenigstens einem weiteren ätherischen Öl gebildet ist.

8. Zusammensetzung nach einem der Ansprüche 1 bis 7, **dadurch gekennzeichnet, dass** das pflanzliche Öl ausgewählt ist aus der Gruppe Palmöl, Sojaöl, Rapsöl, Sonnenblumenöl, Hanföl, Palmkernöl, Baumwollsamenöl, Erdnussöl, Maiskeimöl, Kokosnussöl, Olivenöl, Sesamöl, Leinöl oder Diestelöl, Pinienkernöl, Haselnussöl, Macadamiaöl, Pekanussöl, Walnussöl, Kürbiskernöl, Mandelkernöl, Traubenkernöl.

9. Zusammensetzung nach einem der Ansprüche 1 bis 8, **dadurch gekennzeichnet, dass** die wenigstens eine Fraktion gebildet ist aus kurzkettigen Triglyceriden, mittelkettigen Triglyceriden und/oder langkettigen Triglyceriden.

10. Zusammensetzung nach einem der Ansprüche 1 bis 9, **dadurch gekennzeichnet, dass** das ätherische Öl Terpene, insbesondere Monoterpene und/oder Sesquiterpene, und/oder aromatische Verbindungen aufweist.

11. Zusammensetzung nach einem der Ansprüche 1 bis 10, **dadurch gekennzeichnet, dass** das ätherische Öl ausgewählt ist aus der Gruppe Eukalyptusöl, Teebaumöl, Rosmarinöl, Thymianöl, Pfefferminzöl, Lavendelöl, Kampferöl, Kamillenöl, vorzugsweise auf Basis Römischer Kamille oder Echter Kamille, Grapefruitöl, Bergamotteöl, Weihrauchöl, Majoranöl, Orangenöl, Mandarinenöl, Grün-Minzöl, Ingweröl, Pfefferöl, Zitronenöl, Limettenöl, Basilikumöl, Pinienöl, Bittermandelöl, ätherisches Hanföl, Kümmelöl, Kurkumaöl, Rosenöl, Jasminöl, Fenchelöl, Anisöl, Kakaobohnenöl, Myrrheöl.

12. Vorrichtung zur Verdampfung von cannabidiolhaltigen Zusammensetzungen, vorzugsweise einer Zusammensetzung gemäß einem der Ansprüche 1 bis 11, mit einem Energiespeicher und/oder einem elektrischen Anschluss an eine externe Stromquelle, einem Vorratsbehälter zur Aufnahme der Zusammensetzung, einer Verdampfungseinrichtung zur Verdampfung der Zusammensetzung und einer Applikationseinheit zur Abgabe der verdampften Zusammensetzung an einen Benutzer, wobei zwischen Verdampfungseinrichtung und Applikationseinheit ein Strömungsweg für die verdampfte Zusammensetzung gebildet ist, **gekennzeichnet durch** einen Sensor, wobei der Sensor im Strömungsweg angeordnet und dazu ausgebildet ist, wenigstens einen auf die verdampfte Zusammensetzung bezogenen physikalischen und/oder chemischen Parameter zu sensieren.

13. Vorrichtung nach Anspruch 12, **gekennzeichnet durch** eine Rechnereinheit und eine Sendeeinrichtung zur kabellosen Datenübertragung, wobei die Rechnereinheit mit dem Sensor und der Sendeeinrichtung datentechnisch verbunden ist und dazu ausgebildet ist, Sensordaten datentechnisch zu verarbeiten und die derart verarbeiteten Sensordaten an die Sendeeinrichtung zum Zwecke der Übertragung an eine korrespondierende Empfangseinrichtung weiterzuleiten.

14. Vorrichtung nach Anspruch 13, **gekennzeichnet durch** eine Desinfektionseinheit zur Desinfizierung der Applikationseinheit mittels UV-Strahlung.

15. Verwendung einer Zusammensetzung gemäß einem der Ansprüche 1 bis 11 zur Herstellung eines Inhalats mittels einer Vorrichtung gemäß einem der Ansprüche 12 bis 14.
